(19) **Europäisches Patentamt**
**European Patent Office**
**Office européen des brevets**

(11) **EP 4 778 572 A1**

(12) **EUROPEAN PATENT APPLICATION**

(43) Date of publication:
**22.07.2026 Bulletin 2026/30**

(21) Application number: **25315018.9**

(22) Date of filing: **21.01.2025**

(51) International Patent Classification (IPC):
*A61N 7/02* (2006.01)   *A61B 34/00* (2016.01)
*A61B 8/00* (2006.01)   *A61B 90/00* (2016.01)
*A61N 7/00* (2006.01)   *A61B 34/10* (2016.01)

(52) Cooperative Patent Classification (CPC):
**A61N 7/02; A61B 8/085; A61B 8/4218;**
**A61B 34/25; A61B 90/37;** A61B 34/30;
A61B 2034/101; A61B 2034/107; A61B 2090/378;
A61N 2007/0034; A61N 2007/0091

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Theraclion**
**92240 Malakoff (FR)**

(72) Inventors:
• **GEROLD, Björn**
**78000 Versailles (FR)**
• **BERTHOUMIEUX, Lena**
**75017 Paris (FR)**
• **ANQUEZ. Jérémie**
**75013 Paris (FR)**
• **GRISEY, Anthony**
**78210 Saint-Cyr-L' Ecole (FR)**

(74) Representative: **Hepp Wenger Ryffel AG**
**Friedtalweg 5**
**9500 Wil (CH)**

(54) **A HITU DEVICE**

(57)    The invention is directed to a HITU device (200) comprising a therapy transducer (202), a displacer (205) for the therapy transducer (202), and a controller (211), and preferably a RF generator (208). The displacer (201) is adapted to move the therapy transducer (202) such that a focus (F) of the therapy transducer (202) is moved in a first direction (215). The controller (211) is adapted to operate the displacer (205) to move the therapy transducer (205) while the therapy transducer (205) is emitting a pulse (P).

Fig. 16

**Description**

The present invention relates to a HITU device and associated methods according to the preamble of the independent claims.

Ultrasonic waves are known in the prior art as a means for therapy and imaging.

WO 2020/254419 A1 discloses a method of performing a heat treatment of blood vessels, such as varicose veins, in order to coagulate the vessel walls. Bubbles are created in the blood, such that the formation of blood coagula is avoided during the treatment due to cavitation.

US 2017/0151448 A1 discloses a device for therapeutic treatment which comprises a HIFU transducer for generating and transmitting HIFU pulses to a target. The HIFU transducer is operable in at least a probing mode and in a treatment mode.

US 9,770,605 B2 discloses a method for treating a desired volume of tissue using HIFU or other energy modality includes ablating a pattern of elemental treatment volumes each having a volume that is greater than that of the focal zone of the HIFU transducer but smaller than the overall volume of the desired treatment volume.

N Barnat et al. (Noninvasive vascular occlusion with HIFU for venous insufficiency treatment: preclinical feasibility experience in rabbits. Physics in Medicine and Biology, 2019, 64 (2), pp.025003. doi:10.1088/1361-6560/aaf58d. hal-02403722) discloses a study on the potential of ultrasound exposures in continuous wave mode to thermally occlude rabbit saphenous veins.

In known therapeutic applications, e.g. in imaging and High-Intensity Therapeutic Ultrasound (HITU) such as High Intensity Focused Ultrasound (HIFU), pulses are delivered and cause a lesion to a target tissue. Heat deposition and lesion creation/growth is generally favored by cavitation/boiling, but reaching this regime may require long pulse duration and/or high power.. Creating a thermal lesion often requires a substantial thermal energy to be deposited. In this regime, the balance between power and duration is hard to determine due to local and inter-individual variations in the tissue properties and geometry. Indeed, excessive power carries the risk of inducing skin burns, while a insufficient power may prohibitively increase time to create the lesion. There is a need in the art to provide device and methods which allow safely creating thermal lesions while limiting treatment duration.

This and other objects are achieved by the devices and methods according to the characterizing portion of the independent claims of the invention.

The invention is directed to a HITU device which comprises a therapy transducer, a displacer for the therapy transducer (e.g. a robotic arm), and a controller. Optionally, the device also comprises a Radio Frequency (RF) generator. The displacer is adapted to move the therapy transducer such that a focus of the therapy transducer is moved in a first direction. Furthermore, the controller is adapted to operate the displacer to move the therapy transducer while the therapy transducer is emitting a pulse.

Thereby, the HITU device is adapted to deliver HITU pulses with an ultrasound transducer during which the focus is moved along a line.

In particular, when a pulsed mode is used, lesions may be created very fast. However, the lesion is created in a vertical direction towards the transducer due to reflection of the HITU beam by a bubble cloud. For targets whose shape is at least partially flat (e.g. a collapsed vein), it may thus be necessary to restrict pulse durations to very short times (e.g. few tens of milliseconds) and interrupt the treatment and move to another location.

By moving the focus continuously at a speed which results in a movement of the focus corresponding to a focus size (or less) in the time needed to reach cavitation/boiling), sufficient therapeutic results may be achieved while avoiding skin burns. Thus, treatment speed is increased while continuously being in a cavitation/boiling regime, which results in a very efficient treatment while avoiding lesion overgrowing.

The ultrasound transducer is preferably a piezoelectric transducer (e.g. a piezoceramic transducer or a piezocomposite transducer) and is mounted on a HITU treatment head. The device may be equipped with a RF generator able to deliver a high-power radiofrequency signal. In a preferred embodiment, the treatment head is mounted on a robot which enables controlled movements.

In a preferred embodiment, the controller is adapted to send a command to the RF generator to activate the delivery of electrical energy. The RF generator may then deliver electrical energy to the therapy transducer. The therapy transducer translates this electrical energy into acoustic energy. At the same time, the controller instructs the therapy transducer movement system to move.

Due to the displacement of the HITU treatment head, the geometrical focus of the focalised ultrasound delivered by the piezoelectric transducer may be moved along a line.

For example, if the target is a vein (which may be collapsed), the target has a limited thickness (typically 0-2 mm). Thus, moving the focus along a line allows to decrease the height of the thermal lesion, thus, to limit thermal damages to adjacent tissue while heating the whole diameter of the vein in one or a limited number of pulses. Thereby, the duration of the treatment may also be decreased.

Moreover, the fact to move away from the initial position of the beam during the pulse may spread the energy at a skin level, thus enabling higher power levels and/or decreasing the need for interpulse cooling time.

Therefore, it may also be possible to emit pulses at a power which is sufficient to substantially instantaneously induce boiling. Instead of, growing a lesion vertically towards the transducer (as would be the result with a static pulse known in the art), moving the focus laterally is results in a flatter lesion which corresponds to the compressed vein geometry. Thus, heat diffusion may not be a significant factor in defining lesion size and/or geometry.

Preferably, the controller is adapted to operate the displacer to move the therapy transducer along a linear path.

Preferably, the pulse is pulsed according to a predefined duty cycle and pulse repetition frequency.

A pulsed mode may enable to use high power sufficient to reach cavitation/boiling quickly while avoiding skin burns. A pulsed mode allows to increase the power while limiting the energy delivered to the same level.

In general, the high-power radiofrequency signal generated by the RF generator may be pulsed (i.e. the signal amplitude is not constant, but the amplitude varies preferably periodically during the pulse).

It is advantageous to use pulsed pulses because, at the same mean energy as a continuous pulse, higher power levels may be used during bursts. Thereby, nonlinear effects are increased which favors heat deposition at focus as opposed to outside the focus. This may increase heat deposition at focus and enable reaching cavitation boiling quickly while increasing patient safety and avoiding skin lesions/burns.

When combined with a continuous linear focus movement, a synergetic effect may arise since overgrowing of the lesions towards the transducer in the cavitation/boiling regime may be avoided. A possible alternative solution would be to deliver a short pulsed pulse, then to move to a next spot and repeat, but it was found that, surprisingly, the advantages of the pulsed regime are maintained when the focus is moved at a speed in the order of a few mm/s or cm/s while avoiding a stop-and-go motion of the displacer/robot/treatment head (which is detrimental to the mechanical pieces and causes wear and tear, and further challenging to implement). Continuous focus movement imay include that no sub-pulses within a pulse are delivered in a static setting.

Moreover, the frequency associated with the pulse repetition frequency (PRF) may be lower than a sinusoidal frequency used during the bursts, which may promote cavitation, which in turns may increase heat deposition at focus.

Several types of pulsed signal may be used. For example, the RF generator may generate a signal modulated by an envelope of a square-wave signal for which the amplitude of the signal, the frequency repetition of the pulse and the duty cycle are constant. The RF generator may also generate a signal with amplitude modulation based on square signal of variable amplitude. The duty cycle may also be different during a pulse. The RF generator may also generate envelopes other than a square-wave signal, for example, following a sinusoid or any other type of envelope.

In a preferred embodiment, the controller is adapted to cause the HITU device to deliver pulses with at least one of the following set of parameters:

- for a pulse frequency within 0.8 MHz and 1.2 MHz, an advantageous reference domain of parameters is defined by a duty cycle between 20% and 100%, an acoustic power between 500 W and 1000 W and the focus is moved automatically (e.g. by the displacer, robotically or using beam steering along a linear trajectory) at a speed of 5 mm/s. These parameters are particularly advantageous for a focus depth between about 25 mm and 38 mm under the skin;

- for a pulse frequency within 0.8 MHz and 1.2 MHz, an advantageous reference domain of parameters is defined by a duty cycle of 20%, an acoustic power between 370 W and 850 W and the focus is moved automatically (e.g. by the displacer, robotically or using beam steering along a linear trajectory) at a speed of 5 mm/s. These parameters are particularly advantageous for a focus depth between about 15 mm and 25 mm under the skin;

- for a pulse frequency within 2.5 MHz and 3.5 MHz, an advantageous reference domain of parameters is defined by a duty cycle of 20%, an acoustic power between 60 W and 365 W, and the focus is moved automatically (e.g. by the displacer, robotically or using beam steering along a linear trajectory) at a speed of 3 mm/s. These parameters are particularly advantageous for a focus depth of about 5 to 14 mm under the skin.

As known by the skilled person, these advantageous depth ranges may slightly vary (by a few mm) depending on the exact transducer geometry (f-number etc.) and the nature of the targeted tissues.

It will be understood that during a treatment, the whole parameter range may not be accessible to the user (e.g. due to hardware limitations).

Around these sets of parameters, similarly advantageous sets of parameters may be derived as follows:

Relationship between movement speed (s), duty cycle (d) and acoustic power ($P_{ac}$) at constant depth:

Pulse parameters are considered substantially equivalent if the movement speed, the pulse power and the PRF are varied according to the following law:

$$P_{ac} \times \frac{d}{s} = constant$$

Dividing the acoustic power by 1.2 can be compensated by multiplying duty cycle by 1.2 to deliver substantially the same energy to the tissue, or by dividing the movement speed by a factor 1.2 or a combination of both.

Using the above, it is possible to translate advantageous parameters to an alternative preferred domain of parameters, e.g. if practical considerations prohibit delivery of pulses with the originally intended parameters.

For example, acoustic power may be limited by the capacity of the RF generator. The above formula neglects effects related to nonlinear propagation and may be further refined by adding an exponent to the P_ac term. If the movement speed is modified, the pulse duration may be modified accordingly to maintain the same lesion size (e.g. if the movement speed is divided by two, the duration of the pulse may be multiplied by two). Alternatively, the pulse duration can be kept constant, which may be advantageous to handle pain. Indeed, pain sensation increases with the duration of the pulse.

Equivalence when changing depth: a derating formula can be used to derive substantially equivalent pulse power at other depths. Typically, with the focus at a depth under skin $z$, and a frequency $f$ in MHz, the derated power $P_d$ can be computed as follows:

$$P_d = P_{ac} \times \exp(2 \times f \times \alpha \times z)$$

with $\alpha$ an attenuation coefficient preferably comprised between 0.005 and 0.02 Np/mm/MHz (most preferably between 0.005 and 0.01 Np/mm/MHz). This enables to extrapolate the reference parameters domain to other depths.

Depth under skin may be understood as a thickness of the tissue layer between the transducer and the target and/or focus. It may for example be measured based on an automatic skin detection algorithm on a monitoring image (e.g. a live B-mode image). For example, it may be measured at the center of the monitoring image. For example, it may be the distance between the skin and the geometrical focus, or an estimation of the actual focus (e.g. obtained by numerical simulation), or another point related to the expected lesion (e.g. the center of the expected lesion along the main ultrasound propagation axis).

In a preferred embodiment, the reference parameters domain (or the reference parameters domain extrapolated to another depth) may be limited by a maximum allowed power (or a maximum duty cycle or a minimum speed or a maximum pulse duration) to limit skin exposure to tolerable levels. For example, if the spatial peak and temporal peak of the acoustic intensity at skin level (Isptp) within the impinged skin area is limited, it may reduce the extrapolated reference parameters domain at certain depths depending on the shape of the acoustic field. Note that Isptp at the depth of interest may be computed based on measurements for each transducer or be computed by a general formula considered valid for all transducers. "Peak" may be understood as a value characterizing a thermally relevant level of exposure (e.g. the maximum intensity after a certain transformation (e.g. a gaussian blurring) is applied to the intensity field, or a quantile). Similarly, geometry of the impinged skin zone may be approximated (e.g. by a plane orthogonal to the main ultrasound propagation axis or based on 2D image) or fully determined in 3D (e.g. by rotation an ultrasound transducer around the main ultrasound propagation axis).

The pulse repetition frequency (PRF) is preferably chosen so that at least 3 repetitions (most preferably at least 10 repetitions) occur when the focus moves by a distance corresponding to the size of the -6 dB focal spot. For example, if the focal spot size is 1.5 mm along the X axis and the focus moves along this direction with a speed of 5 mm/s, the pulse repetition frequency is preferably above 10 Hz (most preferably above 33 Hz). Preferably, the PRF is defined so that at least 5 periods of the acoustic wave (most preferably at least 100 periods) are contained in a repetition.

Pulse duration is preferably limited to 8 seconds (most preferably to 4 seconds, further preferably to 1 second), but preferably greater than the size of the focal spot along the movement direction divided by the movement speed. It may vary from one pulse to another (e.g. based on the local diameter of the vein or maximum tolerated skin exposure at a given depth).

Inter-pulse pauses are preferably introduced between pulses.

This is advantageous to limit pain sensation which tends to increase with pulse duration and to avoid skin overheating. These pauses may be of constant duration or their duration may be defined to be as short as possible based on the pulse parameters used but long enough to allow skin to cool down before the next pulse. In the latter case, the Intensity at skin level (either peak or averaged over the impinged skin area) averaged in time over the pulse and subsequent pause time may for example be kept under a certain threshold.

Limitations in depth may also be imposed for each pulse in the form of user information when the depth under skin is outside the recommended range, and/or automatic pulse type switching.

In a preferred embodiment, the HITU device is able to deliver pulses in both described reference parameters domain (or substantially equivalent domains as per the formulas above), preferably using a transducer (preferably a piezo ceramics) at its resonance frequency f_0 and third harmonics 3×f_0. Preferably, the maximum range of parameters accessible to the user (and/or by an embedded software which would automatically select or propose the appropriate pulse parameters) enables to make the derated power (or equivalent parameters as per the above formulas) vary by at least 10% (most preferably at least 15%). This is advantageous because it enables to adapt the pulse parameters during treatment depending on tissue parameters or on tissular response (e.g. occurrence or size of hyperechoic marks). In a preferred embodiment, the f-number of the HITU transducer (or equivalent f-number of the transducer array taking into account beam steering) is smaller than 0.8, most preferably smaller than 0.7. This is advantageous because it limits overgrowing of the lesion towards the transducer for parameters within the described domain of parameters.

It will be understood that the HITU device and in particular the controller may be adapted to deliver pulses (e.g. by causing the displacer to move and the transducer to emit pulses accordingly) with any of the above characteristics.

The HITU device may comprise a user interface.

Preferably, the user interface is adapted to show a representation of an expected impinged tissue area.

The HITU device may be equipped with an imaging transducer (separate from or combined with the therapy transducer) which provides real-time ultrasound images of the treated area and it allows to have a representation of the impinged tissue area.

The user may then identify the impinged tissue area and determine if it is necessary to modify the characteristics of the pulse. For example, if the pulse trajectory length is 10 mm, the expected size of the impinged area for the treatment to be considered effective may be plotted on the screen. For example, its length may be 10 mm, or more if heat diffusion is expected, or less, e.g. if tissue dragging is expected to occur.

The location of the impinged tissue area along the main ultrasound propagation axis may differ in relation to the focus for different pulse parameters.

The HITU device, in particular the controller, may further be adapted to determine a central line of the vein. For example, a rotation of am imaging device may be performed to find the longitudinal view of the vein, and/or automatically detect/enable the user to point out the location of the vein in several transverse slices, thus defining the central line of the vein.

Preferably, the HITU device comprises a software code adapted to compute the expected impinged area based on a skin position relative to the focus, and/or based on a skin position relative to the therapy transducer, and/or based on a location of a hyperechoic marks of a previous pulse.

In a preferred embodiment, the expected impinged area is computed based on the skin position relative to the geometrical focus and/or the transducer. The therapeutic transducer may be curved to obtain a geometric focal point defined by its radius of curvature.

It is advantageous for the user because to visualize the impinged zone to precisely target the vein and to avoid unnecessary damages to surrounding tissue.

Absent refraction of ultrasound beam on the different surfaces it passes through during a pulse, the focal point would be at a distance d from the transducer. The therapeutic ultrasound beam may pass through different tissues which may be heterogeneous and have different mechanical characteristics, thereby inducing refraction and distortion of the beam. In the context of the present disclosure, a single HIFU beam may be used phased array transducers are also conceivable.

To analyse the impact of the refraction of therapeutic ultrasonic beam on the location of the focal point, Snell's law may be used. The velocity of the ultrasonic beam in water is denominated $v_1$ and the velocity in inhomogeneous tissues is denominated $v_2$. $v_1$ may be known while $v_2$ may need to be determined experimentally. $O_1$ is the angle between the high-intensity ultrasound rays and the skin in the water and $O_2$ in the inhomogeneous tissues.

$$Snell's\ law: \frac{\sin(O_1)}{\sin(O_2)} = \frac{v_1}{v_2}$$

With the characteristics of the transducer, some trigonometric relations and Thales' theorem may be used to find the distance between the skin and the focal point. Alternatively, the dependence of the location of the targeted zone with the skin position may be experimentally determined (e.g. by analysing the location of hyperechoic marks during in vivo experiments) and/or adjusted during the course of the treatment (e.g. based on an automatic algorithm segmenting the hyperechoic marks). It may also depend on the location of hyperechoic marks observed during previous pulses delivered at a neighbouring location (e.g. on the same transverse slice of the vein). Indeed, hyperechoic marks may reflect the presence of bubble clouds which reflect the ultrasound beam, thus increasing heat deposition upstream and shielding the tissue located further away from the transducer.

Refraction and tissue heterogeneity may also impact the imaging beam and possibly distorting the image. Indeed, the parameters of the imaging transducer are set by the manufacturer on the assumption that ultrasound only passes through biological tissue.

The user interface may be adapted to allow a user to define a trajectory length.

For example, the user may observe the area to be treated on the user interface and then define the length of the trajectory, e.g. with a slider. If the target is a vein, and if the motion of the focus is done orthogonally to the main axis of the vein, the trajectory length would typically be chosen to be the diameter of the vein. Such a user interface is advantageous because it may allow the area to be treated to be precisely targeted and protects the perivenous tissues.

In a preferred embodiment, the maximal trajectory length is defined by the size of the ultrasound image. The target preferably remains visible on the screen throughout the pulse to allow user control. The skin through which the ultrasound passes may preferably be cooled, and the defined trajectory may preferably not extend beyond the area pre-cooled by the system. To this end, the controller may be adapted to determine a cooled area and/or receive said information from a cooling device.

The user interface may be adapted to allow a user to define at least one of a target width, a number of pulses to be performed to cover a target, a duration of the pulse, a movement speed of the focus, and/or a direction of a focus movement, for at least one and/or each one and/or a sequence of pulses.

In a preferred embodiment, the user interface is adapted to allow the definition of one or a sequence of pulses through the total targeted zone length. If only one pulse is delivered (as defined in the pulse sequence), the motion of the focus is done all along the targeted zone length. If a sequence of more than one pulse is delivered, the total targeted zone length may be split. The number of pulses to cover the total length may be defined on the user interface. The user interface may also be used to define the target width, which is the position of the target, which is the position of the area to be treated along the main direction of ultrasound propagation. It is also possible to define the speed at which the transducer moves over the area to be treated during the pulse. The user interface may enable the definition of the duration of the pulse and/or the focus movement's direction.

Only two of the three parameters - trajectory speed, pulse duration and trajectory length - may be needed to define the sequence of pulse. It is also possible to lock the values of certain parameters and authorise the modification of one of the parameters listed above only to allow the definition of one or a sequence of pulses.

It is advantageous to be able to manually modify each of these parameters to adapt the treatment to each patient. For example, if the patient experiences pain during a pulse for which the trajectory length is long, the user may increase the speed of movement of the focus so that the pulse lasts less time. The user may also increase the number of pulses needed to cover the entire target, to reduce the time taken for each shot. The user may also shorten the duration of a pulse.

It is also advantageous because the user may reduce the duration of the treatment by adjusting the parameters.

Preferably, the treatment progression direction of a sequence of pulses is the same as the pulse trajectory direction. For example, if the treatment starts at the left extremity on the veins, the direction of trajectory is from left to right. Thus, if the length of the zone to be targeted is divided into three pulses, the order of the pulses delivered will go from left to right, starting with the leftmost pulse, then the central pulse and then the right-hand pulse. Thereby, the number of displacements between pulses is minimized.

Alternatively, the user has the choice between different pulse delivery orders.

In a preferred embodiment, the user interface is adapted to allow the definition of a pause time between two consecutive pulses.

This parameter may also be locked.

A minimum pause time may be automatically computed based on predefined rules (e.g. based on acoustic intensity at skin level), which the user may increase if needed (e.g. if the patient experience pain or if the user wants to talk to the patient).

It is advantageous to define a pause time between two consecutive pulses to allow the skin to cool down and return to an acceptable temperature for receiving a new dose of ultrasound.

The user interface may be adapted to allow a user to define at least one of a duty cycle of a radiofrequency signal delivered by the RF generator, a pulse repetition frequency of the high-power radiofrequency signal delivered by the RF generator, a movement speed of the focus, an output power of the RF generator, and/or an instantaneous intensity at the skin surface during a pulse delivery.

It is advantageous to modify these parameters because it allows to modify the energy deposition on the target area.

In a preferred embodiment, the user interface is adapted to enable the definition of the duty cycle of the high-power radiofrequency signal delivered by the RF generator. The duty cycle corresponds to the ratio between the duration of ultrasound delivery and the period. The pulse repetition frequency of the high-power radiofrequency signal delivered by the RF generator may also be set. It is advantageous to be able to modify these parameters to increase or decrease the energy delivered on the target area. Typically, if the pulse is not sufficient, the user may increase the value of the duty cycle to deliver more energy during the pulse.

The user interface may also enable the definition of the output power of the RF generator it may be useful to decrease or increase the energy deposition on the target area.

Moreover, the user interface permits the modification of the Intensity Spatial Average Time Peak ($I_{SATA}$) during pulse delivery, the value being understood as $I_{SATA} = \frac{P_{Ac}}{S}$, where $P_{Ac}$ corresponds to the acoustical power emitted by the therapy transducer that will reach the skin surface, and S is the skin area intercepted by the HIFU beam.

By modifying this value, at a constant skin area, the acoustical power will vary. Typically, if the $I_{SATA}$ value increases by 10 W/cm$^2$, the maximal acoustic power that may be delivered by the system will increase.

The user interface also allows the user to modify the movement speed of the focus. Typically, the user may increase the movement speed of the focus, to reduce the linear energy.

The user interface may be adapted to allow a user to define a frequency band of a radiofrequency signal delivered by the RF generator.

In a preferred embodiment, the user interface is adapted to allow the definition of the frequency band of the high-power radiofrequency signal delivered by the RF generator. In a preferred embodiment, the RF generator is adapted to deliver at least two frequencies (f0 the resonance frequency of the therapy transducer and 3f0). This is advantageous to treat target at different depths under skin.

Higher frequencies are more suited to treat superficial targets due to higher absorption, while lower frequencies enable greater penetration, thus to treat deeper targets. It is advantageous to be able to define the frequency band of the high-power radiofrequency signal delivered by the RF generator because, e.g. if the user notices that the pulse does not induce the expected tissular reaction (e.g. hyperechoic marks), he may change the frequency band to treat the target.

The HITU device may comprise means for synchronizing the emission of the pulse and motion, e.g. the trajectory, of the focus.

The means for synchronizing may be formed by a cable, where the RF generator and/or the displacer are adapted to send and/or receive data. Alternatively, the means for synchronizing may be formed by a dedicated control unit which receives data and/or determines parameters of RF generator and/or displacer and coordinates their operation.

The means for synchronizing may be adapted such that smaller equivalent power is emitted (or emission is stopped) when the motion is decelerated.

For example, the controller may send a command to the displacer (e.g. robot) to start the trajectory. In parallel, the controller may send a command to the RF generator to start delivering the pulse. The robot sends position, speed and/or acceleration data to the controller. When the controller receives a negative acceleration data or calculate a negative acceleration with the position or the speed of the robot, it sends a command to the generator to stop pulsing. Alternatively, the expected start time of the deceleration phase is known with enough precision to set the appropriate duration to the pulse beforehand.

It may be advantageous to not deliver energy during the deceleration phase of the trajectory to decrease skin heating. Indeed, transducer movement enables to average the heating effect of the potential acoustic hotspots at skin level. The maximum power that may be delivered is limited by an indicator which ensures that no damage is caused to the patient's skin. This indicator is determined for a pulse delivered at a given minimum speed.

During the deceleration phase of the trajectory, the speed at which the focal point moves become lower than the minimum speed at which the indicator was found, which may increase skin heating.

Moreover, it may be advantageous because during the deceleration phase of the trajectory, more energy accumulates at the focal point. There is therefore a risk that the bubbles created by the accumulation of energy will grow towards the transducer and create damage above the target.

The means for synchronizing are adapted such that smaller equivalent power is emitted (or emission is stopped) when the motion is accelerated.

To substantially maintain the deposited energy per mm of trajectory, the equivalent power is preferably reduced (or the emission is stopped) during acceleration phase. To reduce the emission during the acceleration phase, it is possible, for example, to substantially reduce the amplitude of the signal during this phase or the duty cycle or the pulse repetition frequency in order to deliver less power during this phase.

In another configuration, the controller may send a command to the robot to perform a trajectory. At the same time, it may send a command to the generator to deliver a pulse and give it a delay before delivering the pulse. The delay is configured to correspond to the system's acceleration phase.

Similarly, it is advantageous to not deliver energy during the acceleration phase of the trajectory to protect the skin.

The HITU device may further comprise a communication system adapted to send data from the displacer to the RF generator or vice versa.

In a preferred embodiment, the synchronization of the pulse emission and movement of the focal point is realised using a communication system between the robot and the RF generator.

For example, the controller may send the parameters of the pulses to the RF generator and to the displacer/robot. The controller may then send a signal authorising the pulse to be delivered. The displacer may start moving and send commands directly to the RF generator, depending on its position. When the displacer detects that it has finished its acceleration phase, for example when its speed becomes stable, it sends the command to deliver a pulse to the RF generator. Similarly, when the displacer detects that it is beginning its deceleration phase, for example that its speed is decreasing, it sends the command to stop the pulse to the RF generator.

Synchronizing pulse emission and movement of the focal point using a communication system between the displacer and the RF generator may allow direct control of the RF generator by the displacer and/or vice versa.

The HITU device may further be adapted to automatically select, based on a selected depth under skin (and preferably taking into consideration at least one other parameter), at least one parameter selected from the following group comprising a movement speed of the focus, a duty cycle of the high-power radiofrequency signal, a pulse repetition frequency of the high-power radiofrequency signal, a frequence band of the high-power radiofrequency signal, a output power of the RF generator, a instantaneous intensity at the skin surface during pulse delivery, pulse duration, and/or a location of the focus, in particular with respect to the main ultrasound propagation axis.

For example, possible criteria for automatic detection may be:

- If no/insufficient tissular reaction: decrease movement speed, and/or increase duty cycle, and/or increase PRF, and/or change frequency band (to a lower frequency if deep, to a higher frequency is superficial), and/or increase power, and/or increase instantaneous intensity.
- If HEM too big/too superficial, and/or occurrence of pain, do the opposite.
- If pain occurs, decrease pulse duration,
- If HEM is too deep or too superficial with respect to the targeted zone, adapt location of the focus accordingly,
- If the vein is going superficial (i.e. closer to the skin), increase frequency.

In a preferred embodiment, different parameters are adapted automatically based on the depth under skin.

To optimise treatment safety and efficacy, limit parameters may be set according to the depth of the HITU focal point under the skin. A minimum movement speed of the focus for the treatment to be effective without burning the skin may be determined according to the depth under the skin.

A duty cycle and a maximum pulse repetition frequency may be set according to the depth under the skin, which limits energy deposition so as not to cause lesions.

At a certain depth under the skin, the frequency of the high-power radiofrequency signal may be switched. Typically, for a given embodiment, to treat the vein superficially (for example, depth under skin between smaller than 14 mm), it may be more effective to deliver a pulse at a frequency of 3f0. To treat a deeper part of the vein (for example veins at a depth under skin greater than 15 mm), the treatment may be more effective with a pulse delivered at the frequency f0. Then, when the depth under skin (which may be automatically calculated by the controller), goes from the superficial to the deep part of the vein (for example, to 14 mm depth under the skin) 1, the signal frequency band may switch from 3f0 to f0.

The instantaneous intensity at the skin surface during pulse delivery and the output power of the generator are determined based on the depth under the skin. The other parameters being modified, it may necessary to limit the maximum power to be determined according to the surface of skin crossed by ultrasound. Typically, when changing the frequency of the signal, the passage of HITU through the skin may change and it may be necessary to calibrate the output power of the generator and the instantaneous intensity on the surface of the skin during the pulse delivery. In addition, the greater the depth under the skin of the area to be treated, the higher the power of the delivered signal may have to be because there are more tissues to cross before reaching the target.

All these parameters may be fixed or defined by the user. Then, if the parameters set by the user do not present any danger to the tissues surrounding the area to be treated, the pulse delivery may be performed with these parameters. Otherwise, the pulse may not be delivered.

In a preferred embodiment, the depth under the skin is known by the controller. If there is a change in the depth under the skin of the area to be treated, the controller sends the RF generator and the robot the command to change certain parameters. It also changes the user interface settings.

For example, if the depth under the skin of the area to be treated increases from the superficial to the deep part of the vein, around 13 mm for example, the controller may receive new data saying that the depth under the skin is at 13 mm. It may then send a command to the RF generator to change the frequency band on which the signal is delivered, modify the duty cycle or pulse repetition frequency of the signal. The controller may also send the displacer a command to change the speed of motion of the focus on the upcoming pulses. The controller may also change the instantaneous intensity at the skin surface during pulse delivery to block the electrical power demand to the RF generator.

It is advantageous that the parameters are automatically adapted based on the depth under the skin to optimize the treatment and minimize user interactions. In some embodiments, it may be observed that depending on the depth under the skin, some parameters no longer allow the pulses to be effective. It is therefore necessary to modify them. The automatic change allows to continue to treat the vein without having to stop to change all the parameters when the target is at the limit value of depth under the skin. This also saves time and optimizes energy deposition.

The HITU device may further be adapted to automatically select, based on a detected occurrence of a hyperechoic mark and preferably taking into consideration at least one other parameter, at least one parameter selected from the following group comprising a movement speed of the focus, a duty cycle of the high-power radiofrequency signal, a pulse repetition frequency of the high-power radiofrequency signal, a frequence band of the high-power radiofrequency signal, a output power of the RF generator, a instantaneous intensity at the skin surface during pulse delivery, a location of the focus, preferably along the main ultrasound propagation axis.

The occurrence of hyperechoic marks may make it possible to determine the effectiveness of the treatment. Treatment may be considered locally effective when hyperechogenic marks appear on an image of the targeted area, e.g. as displayed on the user interface. Consequently, if the occurrence of these marks is low, the treatment may be considered locally less effective. The settings may then be automatically changed by the system.

Typically, if there are no hyperechoic marks, the RF generator may change the frequency band in order to deliver another signal. Consequently, to this modification, the instantaneous intensity at the skin surface during pulse delivery may have to be modified to ensure the safety of the skin with the pulse at the other frequencies.

If there is no hyperechoic mark, the movement speed of the focus may be decreased. The duty cycle and the pulse repetition frequency may also be automatically increased. The output power of the RF generator may also be increased. All these changes may increase the energy deposition at the focus and promote the appearance of hyperechoic mark.

In a preferred embodiment, the controller is assisted by an artificial intelligence algorithm to identify the presence of hyperechogenic marks. In the event that the algorithm does not locate a mark after a determined pulse delivery iteration number, the controller may send a command to the RF generator to change the frequency band on which the signal is delivered, modify the duty cycle, and/or pulse repetition frequency of the signal.

The controller may also send a command to the displacer to change the speed of movement of the focus on the upcoming pulses. The controller may also change the instantaneous intensity at the skin surface during pulse delivery to block the electrical power demand to the RF generator. It is also possible to change some parameters automatically without changing others. For example, the controller may send a command to the RF generator to increase its output power.

In another configuration, the user may confirm the presence of a hyperechogenic mark on the user interface. In the event of many mark absence occurrences, the controller sends commands to the RF generator and displacer simultaneously.

In another configuration, an imaging step may be synchronized with the pulsed signal to analyse the presence or absence of hyperechogenic marks during treatment. If there is no hyperechogenic mark, the controller may send a command to the generator to increase the power. It is also possible to reduce the speed of movement of the HITU focal point, the controller may send a command to do so to the displacer.

It is advantageous to optimize the number of pulses to be delivered. The sooner the hyperechogenic mark appears, the less energy may need to be accumulated in the same area. This also protects the skin.

Since, with the proposed pulses parameters, cavitation/boiling is expected to happen quickly, adaptation of the pulse parameters may be performed after delivering a very short (preferably < 200 ms, most preferably < 100 ms) "probing" pulse, preferably without moving. If no or insufficient reaction is detected (e.g. by an automatic cavitation detector (e.g. based on HEM detection on image or passive cavitation detection), parameters are modified. If a good reaction is detected, the moving pulse is delivered, preferably with parameters inducing no or lower energy deposition during the beginning of the movement, for example during the acceleration phase, since the zone is already treated by the probing pulse. Devices and methods having a probing phase and a treatment phase are disclosed in EP 3 164 192, which is incorporated herein by reference.

The controller may be adapted to compute a dragging of a treated tissue and further to model a real displacement of the therapy transducer considering the dragging. Preferably, the controller may further set pulse parameters based on the real displacement.

When the displacer moves the treatment head over the patient's skin, the tissue may cause resistance to the treatment head's movement.

For example, if the user sets a targeted zone length at 3 mm, the displacer may effectively move the treatment head by 3 mm but because of tissue dragging the focus may only by 2 mm with respect to the vein.

To compensate this phenomenon, the real displacement of the treatment head may be modelled. For example, the user may set the targeted zone length. The focus may be moved following this instruction. The real displacement length of the focus with respect to the target may be measured and compared to the expected displacement length. Based on this information, the pulse parameters may be adapted in a next treatment position.

For example, if the real trajectory length of the focus with the respect to the vein is 50% of the expected trajectory length, the trajectory length of the next pulse may be increased (e.g. doubled) and the speed may also be doubled. Preferably, at least one of the speed of displacement, the duty cycle, the pulse repetition frequency of the high-power radiofrequency signal, the output power of the RF generator, and/or the trajectory length are adapted.

In another embodiment, before a pulse (preferably before all pulses) is emitted, a pulse trajectory is performed without delivering acoustic energy. This enables the evaluation of the actual displacement and adaptation of pulses parameters.

Information relative to tissue dragging may be aggregated over several pulses (e.g. averaged). This is advantageous to be more robust to outliers.

In another configuration, the imaging step is synchronized with the pulsed signal to analyse the evolution of the displacement of the treatment head. The amplitude-modulated processing signal is delivered by the RF generator, and the displacer begins a movement. During periods when no energy is delivered, an image may be produced. The displacement performed is analyzed and parameters are modified in real time. For example, in an embodiment, the speed of movement of the focus is increased to protect the skin and to compensate the tissue dragging. Another configuration may be to decrease the power delivered by the RF generator or to decrease the duty cycle or to increase the pulse repetition time. In all these configurations, the energy deposition is modulated.

It may be advantageous to compensate the tissue dragging because it avoids having a surplus excess of energy or a lack of energy in areas not treated because of the tissue dragging.

Tissue dragging may affect different layers of tissue differently. For example, the skin may be dragged to a greater extentthan the vein during motion of the treatment head. Thus, the one or several regions of interest used to compute the actual movement performed may be selected to follow a certain phenomenon. For example, they may be selected to monitor the displacement of the skin in order to avoid skin burns. Alternatively, the skin safety parameters may be defined so as to also be safe when the treatment head does not move with respect to the skin. In this case, for example, the actual motion of the treatment head is preferably computed at a depth under skin corresponding to the targeted zone. It is advantageous to maintain adequate energy deposition or focus replacement after a pulse sequence with respect to the target.

The invention is further directed to a method of treating a patient with HITU. The method includes a step of emitting a HITU pulse from a therapy transducer onto a target, preferably a vein, wherein the therapy transducer is moved in a first direction while a pulse is being emitted. Preferably, the method is performed using a device as described herein above.

Because the focus is moved during emission of the pulse, the energy delivered may be higher while still limiting the lesion size in a direction towards (and/or away) from the transducer. Thereby, a plurality of different target geometries may be treated, in particular substantially flat targets such as compressed veins.

The first direction may be contained in a plane containing a central axis of the target or vein.

The first direction may be oriented substantially orthogonally with respect to a main ultrasound propagation axis and/or parallel to the skin of the patient.

The first direction may be oriented substantially parallel to the main ultrasound propagation axis and/or orthogonally to the skin of the patient.

Therefore, the displacement of the HIFU focus may be performed done along a vertical line, i.e. which corresponds to moving the therapy transducer substantially following the main ultrasound propagation axis and/or along an axis which follows the main axis of the vein with a non-null component along the main ultrasound propagation axis.

This is advantageous for treating perforators which connect the superficial system to the deep system and are often have a vertical or oblique path.

In a preferred embodiment, the motion of the focus is done along X axis, which may be the axis orthogonal to the main axis of the vein and orthogonal to the main ultrasound propagation axis. This is especially advantageous if the HITU device comprises an imaging mode providing a transverse view of the vein during treatment since the movement is performed in the imaging plane. Moreover, it is advantageous because the compression field used to collapse the vein typically results in the vein being horizontal.

In a preferred embodiment, the motion of the focus is done along Y axis, the main axis of the vein.

It is advantageous because it allows a combination with an adjustment of the pulses' duration (e.g. depending on the tolerance to pain of the patient). Indeed, when the pulse duration is tuned, it may happen that, for example, the vein has a diameter of 8 mm, but the length covered by a pulse is 6 mm. In such a case, delivering the pulses along X may result in a suboptimal treatment duration, because two pulses would be needed to cover a vein diameter. If the interpulse cooling duration is fixed, it may mean that treating a cross-section would require two pulses and interpulse cooling times. Since the vein length is virtually unlimited along Y (when compared to the length treated by a single pulse), the problem does not occur when the trajectories are along Y. Preferably, in this case, the amplitude of the trajectory is smaller than 2 cm (most preferably smaller than 1 cm). This is advantageous since some regions may be more painful for the patient. Keeping treated sections along the vein short enables to more easily skip painful zones.

If the vein does not stay at a substantially constant Z along the pulse trajectory, the focus is preferably also moved along Z to follow the path of the vein.

In one embodiment, if the HITU treatment head encompasses an ultrasound probe, the ultrasound probe is placed so as to provide a longitudinal view of the vein during the pulses. This is advantageous because it provides a visualization of the trajectory and of the extent of the tissular reaction (e.g. a hyperechoic mark).

In an alternative embodiment, the ultrasound probe is placed so as to provide a transverse view of the vein during the pulses while the trajectory during the pulse is along the vein. This is advantageous because it is more robust to mechanical adjustment of the imaging probe within the treatment head, whichotherwise may cause the vein to be out of plane when rotating the treatment head to a longitudinal view. In this case, the HITU beam is periodically interrupted during the pulse to enable synchronized acquisition of images during the pulse without the blurring of the image induced by the therapy beam. This is advantageous to assess the efficacy of the pulse (e.g. using automatic algorithms detecting the hyperechoic marks during the pulse). With this type of trajectories, it is also easy to skip a painful zone and to resume the treatment a little further.

In another embodiment, if the HITU treatment head encompasses and ultrasound probe, the ultrasound probe may be placed so as to provide a transverse view of the vein during the pulses. This is advantageous because it may provide a better averaging of the heat deposition at skin level. Indeed, e.g. for a transducer with the shape of a spherical cap, the zone with a higher acoustic field at skin level may be roughly parallel to the zone without substantial HITU field located under the imaging probe. Thus, a movement orthogonal to this direction enables the impinged skin zones to cool down during the pulse when the acoustic shadow created by the imaging probe-related hole in the HITU transducer moves above them.

This is especially advantageous if the HITU device comprises an imaging mode providing a longitudinal view of the vein during treatment since the movement is performed in the imaging plane.

In a preferred embodiment, if the vein path is not straight the trajectory of the pulse follows the path of the vein.

In an alternative embodiment, the pulses are delivered along trajectories combining a movement along X and Y. For example, the trajectory may be linear along X from a first border of the vein until it reaches the second border of the vein. At this point, the movement becomes along Y for a certain predefined distance. Then, it moves along X from the second border of the vein until the first border of the vein, where it starts moving along Y etc. until having treated the desired vein section. This is advantageous because it enables delivering the longer pulses the patient can bear even when the associated treated length is greater than the vein diameter. This path can be split in as many pulses as necessary, e.g. depending on patient tolerance (which may also vary locally and as the treatment progresses). Delivering part of the trajectory along X is especially advantageous when a monitoring mean provides a transverse image of the vein because it enables the measurement of the actual movement performed during the pulse and the adjustment of the trajectory parameters to cover the whole vein diameter. It is advantageous because tissue dragging issues may more likely to be present along this direction which is orthogonal to the femur when treating truncal veins in the thigh.

In a preferred embodiment, the motion of the focus is done according to a locally redefined reference point on the vein after each pulse. This is advantageous for treating a vein by depositing a constant energy per surface (i.e. constant number of Joules per $mm^2$).

In a preferred embodiment, the pulses may be delivered in straight lines between the left and right side of the vein wall (plus or minus a margin, if needed) while moving along Y between cross-sections. Where the vein diameter is constant, the treatment slices are preferably equally spaced, and the spacing may be adjusted (e.g. based on patient tolerance to pain) so an entire number of pulses is required to cover the vein diameter. Where the vein diameter changes, the slice spacing preferably varies. E.g. if the vein diameter increases, the spacing between slices preferably decreases so as to maintain a constant energy deposited by $cm^2$ of vein wall (usually denoted as EFE for Endove-nous Fluence Equivalent).

If the vein does not stay at a substantially constant Z along the pulse trajectory, the focus is preferably also moved along Z to follow the path of the vein.

The motion of the focus may be a rotational motion around an acoustic focus.

In a preferred embodiment, the acoustic focus of the transducer or another point representative of the impinged zone (which may depend on the pulse parameters) is used as a point of rotation. The transducer may be displaced around the acoustic focal point. This is advantageous for protecting the treated patient's skin. Rotating the transducer around the area to be treated evens out energy deposition on the skin.

This is also advantageous for depositing more energy on a precise treatment area, avoiding the rise of bubble cloud overgrowing towards the transducers during the boiling phase induced by the treatment. When a hyperechoic mark is created, bubbles form and rise to create a lesion. It is advantageous to prevent the bubbles from creating an oversized lesion.

The motion of the focus may follow a vein border along a targeted segment.

The focus may be moved in the direction of the main axis of the vein and may also follow variations in the position of the area to be treated along the main ultrasound propagation axis. For example, if the target is a great saphenous vein, its depth under skin varies along its course. Typically, it is deeper at the sapheno-femoral junction, then goes closer to the skin. Thus, it is advantageous for the trajectory of the focus to follow this course as precisely as reasonably possible to target the vein, thus maximizing efficacy while limiting damages to surrounding tissues.

Alternatively, it would be possible, for example, to tilt the transducer head (by means of operating the displacer) to obtain an angle in which the position of the zone to be treated along the ultrasound propagation axis is constant over the whole course of the vein.

In a preferred embodiment, the device comprises a balloon (for example a silicon membrane) mounted on the treatment head (for example, as described in US 9,028,471B2, which is incorporated herein by reference) which may ensure acoustic coupling. Preferably, the pressure of the balloon is controlled by the controller which enables to control compressor compression force exerted on the tissue. The pressure in the balloon may be modified to alter the location of the HITU focal point and to follow the ultrasound propagation axis.

In another configuration, the transducer may be a phased array transducer. The position of the HITU focal point may then be changed without moving the HITU treatment.

The method may further comprise at least one, preferably all, of the following steps:

- placing the focus on a center of the vein, or placing the focus on a right or a left extremity of the vein during a targeting phase when the motion of the focus is done along the horizontal axis, wherein left or right may be understood as left or right with respect to an image on which the vein is being observed,

- the focus is moved to a position outside a tissue area to be treated before emission of the pulse, preferably outside each of a sequence of tissue areas to be treated such as to account for an acceleration phase during which no pulse is emitted,

- at the end of the sequence of pulses and/or when the sequence is interrupted, moving the focus back to the center of the vein and/or to an initial position of the focus, and/or remain at a final position of a last motion.

In a preferred embodiment, when the user defines a trajectory length or a sequence of trajectory, the focus may be placed on a center of the target. Before at least one pulse, the focus may be moved to a position outside the tissue area to be treated during the pulse, or outside each of the segments defined in the pulse sequence, in order to account for the acceleration phase during which no pulse is emitted. Preferably, the focus is moved back to the center of the target at the end of the sequence, or preferably if the sequence is interrupted.

Preferably, if at the end of the sequence, the focus is moved back to its initial position, an algorithm (e.g. a registration algorithm computing an optical flow between an image captured before the start of the sequence and an image at the end of the sequence) is used to check that the focus actually came back to its initial position despite tissue dragging. If it is not the case, a robotic motion is preferably performed to actually reach the initial position. This is especially advantageous when the target is difficult to see and/or the user took care to define the treatment progression axis parallel to the main axis of the target, because, after the sequence, the user can move along the treatment progression axis and be confident about the fact that he is at a good starting position to deliver the next pulse sequence, thereby reducing the need for time-consuming position adjustments or actions to locate the target. When tissue modifications (e.g. hyperechoic marks) are expected to occur during the pulse, the image at the expected location of the tissue modifications is preferably not considered suitable for motion estimation, and motion is preferably estimated based on regions of interested located outside this zone.

Similarly, if at the end of the sequence, the focus is not moved back to its initial position but is expected to be in a final position. The actual final position with respect to the expected final position is preferably estimated based on an image processing algorithm and adjusted if needed. In this case, the zone of the image where the tissue modification is expected to occur is preferably not considered for position estimation.

Based on the image processing algorithm, a coefficient characterizing tissue dragging is preferably computed or updated (preferably one coefficient for each displacement direction), and used to modify the setpoint for the subsequent displacements. Coefficient may be computed based on one displacement (e.g. the previous displacement in a given direction) or several displacements. For example, if the actual movements from left to right are found to be 50 % shorter than expected, the movement amplitude may be doubled for the next movement in this direction. If the next movement in this direction occurs during a pulse, other parameters such as the trajectory speed may also be adjusted (preferably automatically, e.g. doubled, while the pulse duration is kept constant) so as to keep the energy deposition at focus and/or at skin at the expected level. Other pulse characteristics may also be adjusted such as power, pulse duration, PRF... It is advantageous to compute different correction coefficients for the different directions because tissue dragging depends on tissue architecture (e.g. it may be greater when moving towards a bone than when moving away from a bone).

It is advantageous so that the entire area to be treated is covered by the HITU. HITUs are not delivered during the robot's acceleration and deceleration phases, so the focus may be shifted away from the area to be treated to take this phase into account.

In a preferred embodiment, when the user defines a targeted zone length or a sequence of trajectory, the focus is placed on the extremity (right or left) of the target. Before at least one pulse, the focus moves to a position outside the tissue area to be treated during the pulse, or outside each of the segments defined in the pulse sequence, in order to account for the acceleration phase during which no pulse is emitted. Preferably, the focus is moved back to the initial position of the target at the end of the sequence, or preferably if the sequence is interrupted. In another configuration, the focus stays in its final position after the pulse.

It is advantageous so that the entire area to be treated is covered by the HITU. HITU may not be delivered during the robot's acceleration and deceleration phases, so the focus may be shifted away from the area to be treated to take this phase into account.

It is advantageous that the focus remains in its final position after the pulse because the user can then move to the next slice to carry out a yaw process and save time by returning the focus to its initial position.

The method may further comprise synchronizing the emission of the pulse and motion of the focus.

The synchronization may be such that a smaller equivalent power is emitted, or the emission is stopped, when the motion is decelerated.

The synchronizing may be such that smaller equivalent power is emitted, or the emission is stopped, when the motion is accelerated.

In the following, the invention is described in detail with reference to the following figures, showing:

Fig. 1a-1c: different types of pulses.

Fig. 2: a 3D representation of a treatment target.

Fig. 3: schematically a vein system.

Fig. 4 schematically a pulse delivery.

Fig. 5: schematically a rotation of a transducer.

Fig. 6: schematically different configurations of a treatment device.

Fig. 7: schematically a user interface.

Fig. 8: schematically a first way of communication.

Fig. 9: schematically how energy deposition may be reduced.

Fig. 10: schematically a second way of communication.

Fig. 11: schematically a first way of synchronization between pulse and movement.

Fig. 12: schematically a first treatment trajectory.

Fig. 13: schematically a second treatment trajectory.

Fig. 14: schematically a top view of a target.

Fig. 15: schematically a second way of synchronization between pulse and movement.

Fig. 16: schematically a device according to the invention.

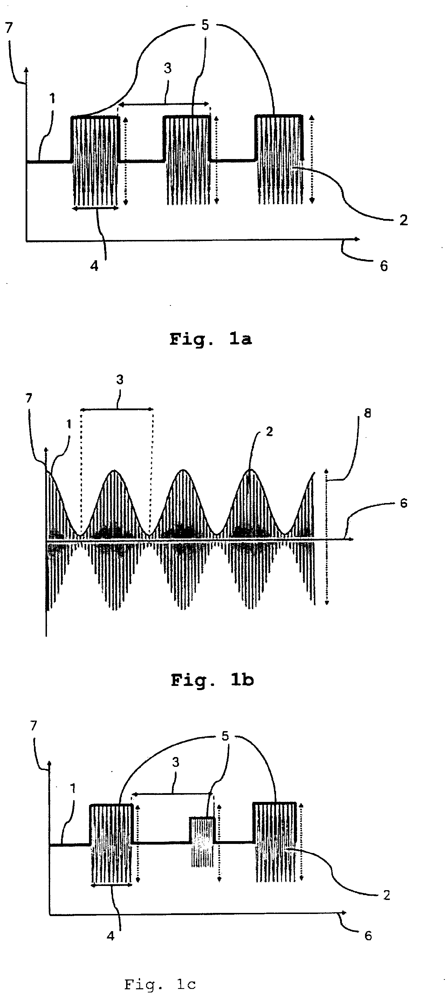

Figs. 1a-1c show non-exhaustive examples of the different types of pulsed signals that may be delivered by an RF generator. The graphs represent the electrical signal delivered by the RF generator 7 as a function of the time 6. The RF generator may generate an envelope signal 1 that may modulate the electrical signal 2 generated by the generator. The shape of the envelope may also be modified. The signal has an amplitude 8, a characteristic 4 representing pulse repetition time multiplied by the duty cycle, and a pulse repetition time 3.

Fig. 1a shows a square-wave signal having an amplitude of the envelope signal 5 which is constant.

Fig. 1b shows a sinusoidal signal.

Fig. 1c shows a square-wave signal having a duty cycle of the envelope 5 which is modified and having a variable amplitude 8 of the signal.

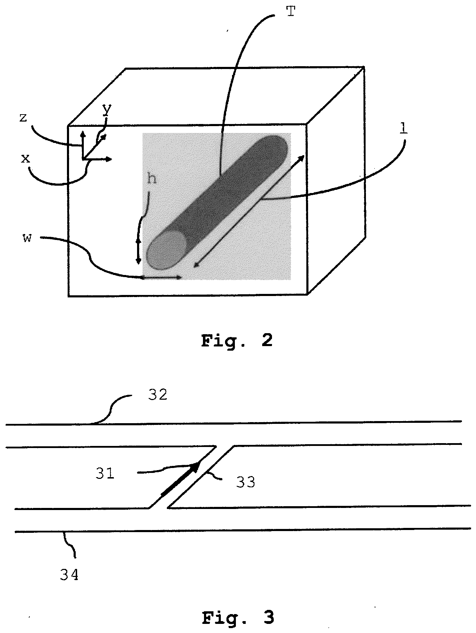

Fig. 2 shows a 3D representation of a treatment target V, in this case a vein. The vein V has a height h, a width w and a length 1. The axis x corresponds to the axis orthogonal to a main axis of the vein V and orthogonal to a main ultrasound propagation axis. Axis y corresponds to the main axis of the vein. Axis z corresponds to the main ultrasound propagation axis. If the vein V is not straight, the axes may be redefined locally, preferably with respect to the vein V and the skin (e.g. along the local main axis of the vein, and/or parallel to the skin) or with respect to the vein and the treatment head (e.g. along the main ultrasound propagation axis, and/or a projection of the local main vein axis in the plane defined by axis x and axis y).

Fig. 3 shows a perforator vein 33 which connects a superficial venous system 32 to a deep venous system 34. The direction of treatment follows an oblique path 31.

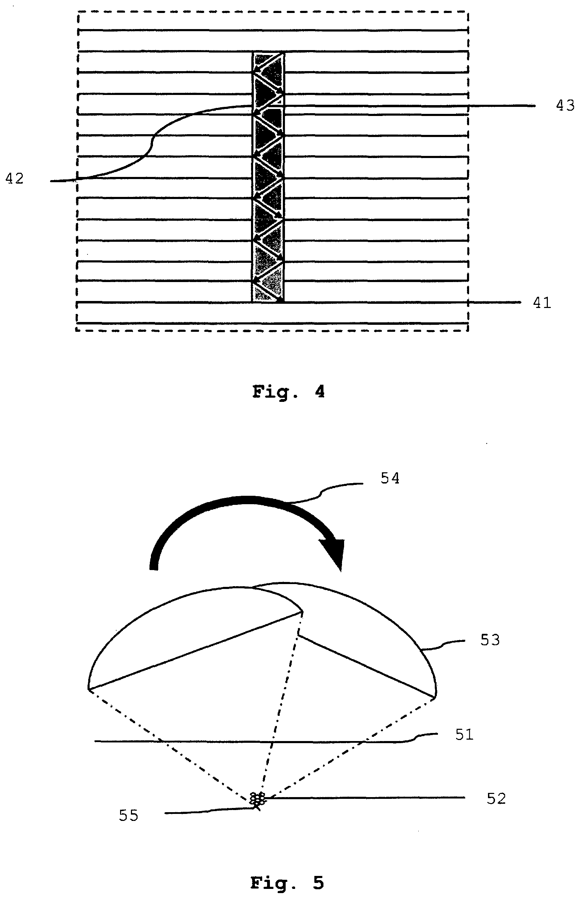

Figure 4 shows an example of pulses delivered along a straight line 43 between edges (on a left and right side) of a vein 42 while moving between cross-sections 41. A top view of the vein 42 is shown. Treatment is delivered along locally-defined straight trajectories 43. Trajectories that may be selected to guarantee constant energy deposition per $mm^2$.

Fig. 5 shows a rotation 54 of a transducer 53 around a focus point 55. A surface area of skin 51 through which the acoustic field passes is variable during transducer rotation, thus harmonizing energy deposition. Bubbles 52 rise as acoustic energy accumulates at a point. The movement of the transducer prevents bubbles from rising.

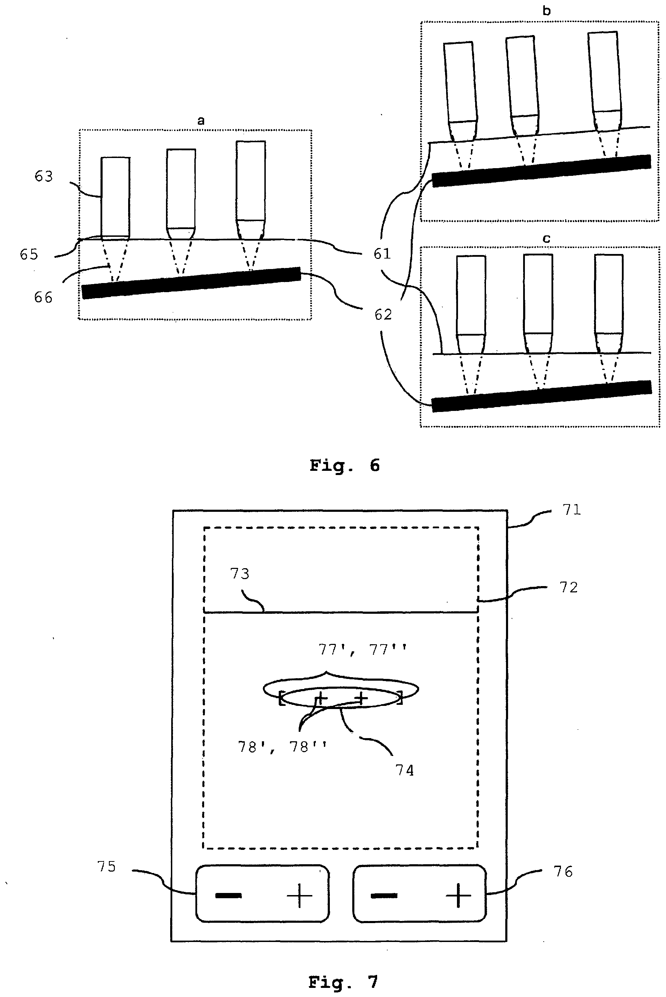

Fig. 6 shows different configurations to ensure that, when a focus is moved in the direction of a main axis of the vein 62 und the skin 61, the focus is also able to follow variations in the position of the area to be treated along the main ultrasound propagation axis. The vein 62 is shown in a longitudinal section. As shown in panel (a), a pressure in a balloon 65 of a visualisation and treatment head 63 is modified so that the HITU beams 66 are focused on the vein 62. To achieve this, when vein 62 is deep, the pressure in balloon 65 is very low and therefore the HITU focal point is deeper. As shown in panel (b), shows the case where the pressure in the balloon 65 is constant, but the treatment head 63 is tilted to obtain a position in which the position of the zone to be treated along the ultrasound propagation axis is constant over the whole of the main axis of the vein. In panel (c), the treatment head 63 comprises a transducer which is a phased array transducer. It is possible to change the depth of the HITU focal point with-out moving the visualization and treatment head 63.

Fig. 7 shows an example of a user interface 71. A collapsed vein 74 and the skin 73 are visible in a B-mode image 72. The user can increase the targeted zone length using button 75 and the number of pulses to be delivered using button 76 to cover the area to be treated. On this example, brackets 77', 77" and crosses 78', 78" represent the start and end of each pulse trajectory. A first pulse is delivered from the left bracket 77' to the left cross 78'. Then a second pulse is delivered from the left cross 78' to the right cross 78". Finally, the third pulse is delivered from the right cross 78" to the right bracket 77'''. Pulses trajectories may be of different lengths and/or pulse parameters may differ within a pulse sequence.

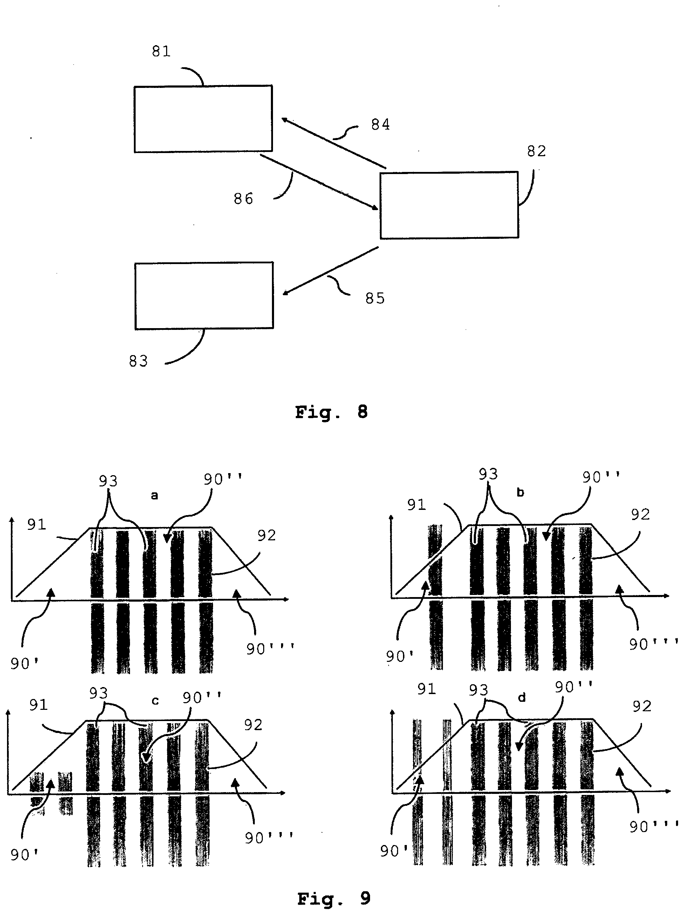

Fig. 8 shows an example of how communication between a controller 82 with a displacer 81 and an RF generator 83 during the deceleration phase of in the motion of a transducer (not shown here) may occur. During this phase, a pulse may not be delivered. In this example, the controller 82 sends a first command 84 to the displacer 81 and a second command 85 to the RF generator 83 to start the movement of the visualization and treatment head (not shown) and the delivery of the high-power radiofrequency signal. The displacer may send a signal 86 comprising position, speed, and/or acceleration data to the controller 82. When the controller 82 receives a negative acceleration data and/or calculates based on speed or the position a negative acceleration data, it sends a command 85 to the RF generator 83 to stop the delivery of the signal.

Fig. 9 shows several examples of how energy deposition per mm may be reduced during the acceleration phase. To reduce the energy deposition per mm, the high-power radiofrequency signal 2 is modulated. Here, the displacer's speed 91 is shown (i.e. speed relative to a position).. The speed profile 91 is divided into an acceleration phase 90', then a stabilisation of speed 90", and finally a deceleration phase 90'''.

As shown in panel (a), no energy 93 may be delivered during the acceleration phase 90'.

Alternatively, as shown in panel (b), by a modification of the pulse repetition frequency, the energy deposition per mm is reduced during the acceleration phase 90'.

Panel (c) shows a reduction of the amplitude of the signal 93 during the acceleration phase 90'.

Panel (d) shows the reduction of the duty cycle of the high-power radiofrequency signal.

All these modifications reduce the energy deposition per mm during the robot's acceleration phase 90'. Similar modifications may be performed during the deceleration phase.

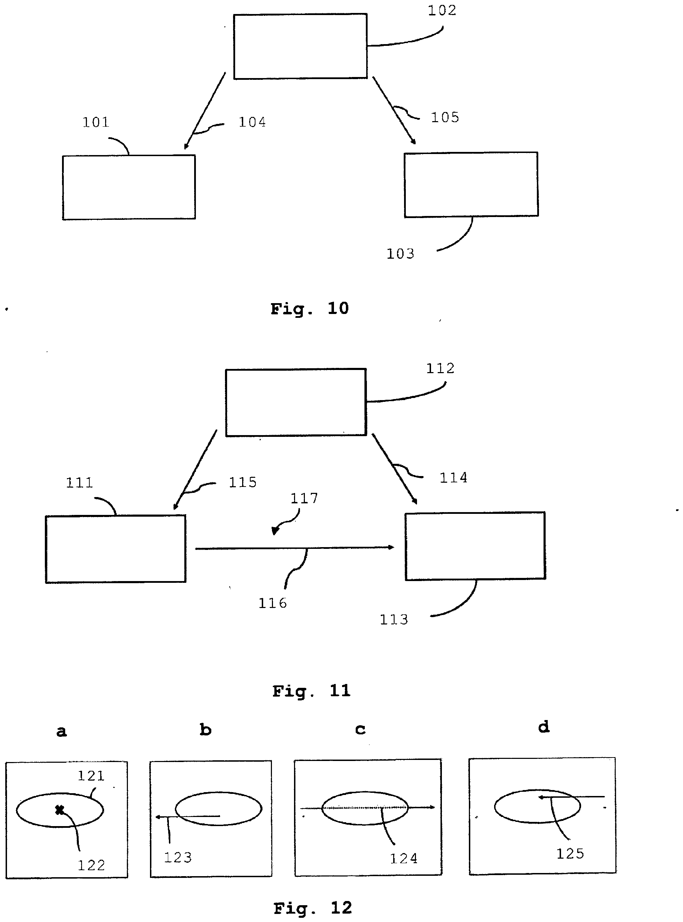

Fig. 10 shows another example of how the communication between the controller 102 with the displacer 101 and the RF generator 103 during the acceleration phase of the trajectory of the displacer. During the acceleration phase, pulse is not delivered (see Fig. 9, panel (a)). In this example, the controller 102 sends a first command 104 to the displacer 101 to start the movement of the treatment and visualisation head (not shown) and, with a controlled delay, a second command 105 to the RF generator 103 to deliver the pulse. The delay corresponds to the system's acceleration phase.

Fig. 11 shows an example of how synchronization between pulse emission and movement of the focal point may be achieved using a communication 117 between the displacer 111 and the RF generator 113. The controller 112 sends a first command 114 at the RF generator 113 with all the parameters needed to deliver the high-power radiofrequency signal. It also sends a second command 115 to the displacer 111 with all the parameters needed to move the treatment head (not shown). Then, the controller 112 sends a signal displacer 111 and RF generator 113 authorizing the delivery of the pulse (through the same channel as commands 114, 115). The displacer 111 initiates motion of the treatment and, once the acceleration phase is over, sends a command 116 directly to the RF generator 113 to start delivering the high-power radiofrequency signal. Then, when the deceleration phase be-gins, the displacer 111 sends a command 116 to the RF generator 113 to stop delivering the pulse.

Fig. 12 shows the trajectory of the treatment head (operated by the displacer) during a pulse in a transverse view. At the beginning of the trajectory, shown in panel (a), the HITU focal point 122 is placed by the user or automatically in the center of the vein 121. Here, the vein 121 is represented partially collapsed but it will be understood that the vein may be fully collapsed or may not be collapsed during treatment.

Then, as shown in panel (b), the displacer causes the treatment head to move to a position 123 outside the vein without delivering a pulse.

As shown in panel (c), the displacer may cause the treatment head to move towards the area to be treated 124. The RF generator (not shown) does not deliver a HITU pulse during the displacer's acceleration or deceleration phases. However, the RF generator does deliver pulses during the phase when the robot's speed is constant. The dotted arrows represent the phases of movement during which a pulse is delivered and the solid arrows represent robotic movement without delivery of a pulse.

As shown in panel (d), the displacer causes the treatment head to the center 125 of the area to be treated.

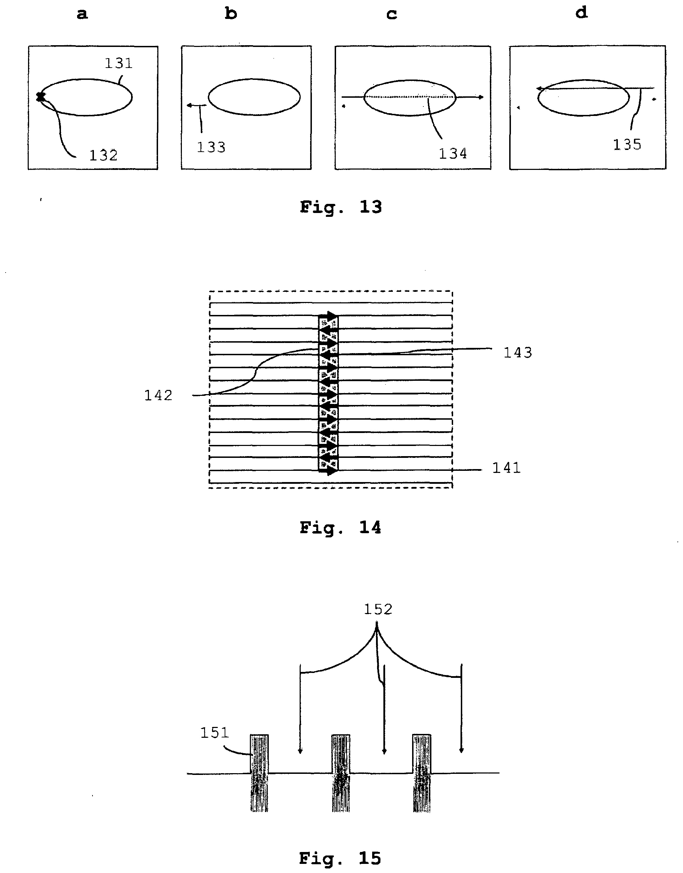

Fig. 13 shows the trajectory of the treatment head (operated by the displacer) during a pulse in a transverse view similar to Fig. 12.

As shown in panel (a), the HITU focal point 132 is placed by the user or automatically in an extremity (in the shown example to the left in the illustration) of the vein 131. Here, the vein 131 is represented partially collapsed but it will be understood that the vein may be fully collapsed or may not be collapsed during treatment.

As shown in panel (b), the displacer causes the treatment head (and thus focus) to move to a position 133 outside the vein 131 without delivering the pulse.

As shown in panel (c), the displacer causes the treatment head to move towards the area to be treated 134. The RF generator does not deliver a HITU pulse during the acceleration or deceleration phases. However, the RF generator does deliver pulses during the phase when the displacer's/treatment head's speed is constant. The dotted arrows represent the phases of movement during which a pulse is delivered, and the solid arrows represent robotic movement without delivery of a pulse.

As shown in panel (d), the displacer causes the treatment head to return to its initial position 125. Another possibility is to finish the movement as in the position shown in panel (c) with-out doing the return shown in panel (d).

Fig. 14 shows schematically a possible treatment. The illustration here shows a vein 142 in a top view and pulse trajectories 143. The user begins the treatment in the first slice 141. Then, the displacer causes the treatment/focus to move from one extremity of the vein to the other extremity (from left to right, or right to left, in the shown illustration), and subsequently the user changes the slice and begins a new pulse without moving back to an initial position. The treatment shown here may be referred to as a lace-up treatment.

Fig. 15 shows an example of the synchronization between the pulsed high-power radiofrequency signal generated by the RF generator 151 and ultrasound imaging 152. Images are taken between each pulsation of the signal.

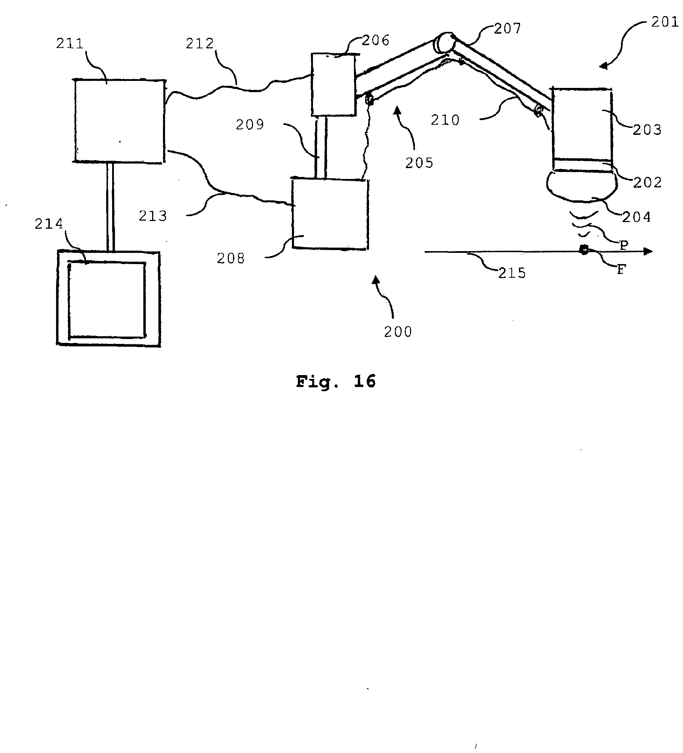

Fig. 16 shows schematically an exemplary embodiment according to the invention. The shown HITU device 200 comprises a therapy transducer 202 embedded in a treatment head 201. The treatment head further comprises a balloon 204 and a housing 203. The transducer 202 has a focus point F to which HIFU pulses P can delivered. The treatment head 201 may be moved by means of displacer unit 205 which is formed by a robotic arm 207 with a displacer control unit 206 which together move the treatment head 201 with the therapy transducer along direction 215. A radiofrequency generator 208 is part of the HITU device 200 and is connected by a data cable 209 forming a communication system to the displacer unit 205. The radiofrequency generator 208 delivers electric radiofrequency signals to the therapy transducer 202 via cable 210. The radiofrequency generator 208 and displacer unit 205 are each connected to a controller 211 via a first cable 212 and a second cable 213. The controller 211 is connected to touch screen 214 forming a user interface through which a user may enter and/or view data and/or view images.

**Claims**

1. A HITU device (200) comprising a therapy transducer (202), a displacer (205) for the therapy transducer (202), and a controller (211), and preferably a RF generator (208), wherein the displacer (201) is adapted to move the therapy transducer (202) such that a focus (F) of the therapy transducer (202) is moved in a first direction (215), and further wherein the controller (211) is adapted to operate the displacer (205) to move the therapy transducer (205) while the therapy transducer (205) is emitting a pulse (P).

2. The HITU device (200) according to claim 1, wherein the pulse is pulsed according to a predefined duty cycle and pulse repetition frequency.

3. The HITU device (200) according to any one of the preceding claims, adapted to deliver the pulse with at least one of the following set of parameters:

   - a pulse frequency between 0.8 MHz and 1.2 MHz, a duty cycle between 20 and 100%, an acoustic power between 500 W and 1000 W, and a velocity of motion of the focus of 5 mm/s, and/or
   - a pulse frequency between 0.8 MHz and 1.2 MHz, a duty cycle of 20%, an acoustic power between 370 W and 850 W, and a velocity of motion of the focus of 5 mm/s, and/or
   - a pulse frequency between 2.5 MHz and 3.5 MHz, a duty cycle of 20 %, an acoustic power between 60 W and 365 W, and a velocity of motion of the focus of 3 mm/s.

4. The HITU device (200) according to any one of the preceding claims, comprising a user interface (214).

5. The HITU device (200) according to claim 4, wherein the user interface (214) is adapted to show a representation of an expected impinged tissue area.

6. The HITU device (200) according to claim 5, comprising a software code adapted to compute the expected impinged area based on a skin position relative to the focus, and/or a skin position relative to the therapy transducer (202), and/or a location of a hyperechoic marks of a previous pulse

7. The HITU device (200) according to any one of the claims 4 to 6, wherein the user interface (214) is adapted to allow a user to define a trajectory length.

8. The HITU device (200) according to any one of the claims 4 to 7, wherein the user interface (214) is adapted to allow a user to define at least one of

   - a target width,
   - a number of pulses to be performed to cover a target,
   - a duration of the pulse,
   - a movement speed of the focus,
   - a direction of a focus movement,

   of one or a sequence of pulses.

9. The HITU device (200) according to any one of claims 4 to 8, wherein the user interface (214) is adapted to allow a user to define a pause time between two consecutive pulses.

10. The HITU device (200) according to any one of the claims 4 to 9, wherein the user interface (214) is adapted to allow a user to define at least one of:

- a duty cycle of a radiofrequency signal delivered by the RF generator,
- a pulse repetition frequency of the high-power radiofrequency signal delivered by the RF generator,
- a movement speed of the focus,
- an output power of the RF generator, and/or
- an instantaneous intensity at the skin surface during a pulse delivery.

11. The HITU device (200) according to any one of the claims 4 to 10, wherein the user interface (214) is adapted to allow a user to define a frequency band of a radiofrequency signal delivered by the RF generator (208).

12. The HITU device according to any one of the preceding claims, further comprising means for synchronizing (206, 208, 209) the emission of the pulse and motion of the focus.

13. The HITU device according to any one of the preceding claims, further comprising a communication system (209) adapted to send data from the displacer (205) to the RF generator (208) or vice versa.

14. The HITU device (200) according to any one of the preceding claims, further adapted to automatically select, based on a selected depth under skin and preferably taking into consideration at least one other parameter, at least one parameter selected from the following group:

- a movement speed of the focus,
- a duty cycle of the high-power radiofrequency signal,
- a pulse repetition frequency of the high-power radiofrequency signal,
- a frequence band of the high-power radiofrequency signal,
- a output power of the RF generator,
- a instantaneous intensity at the skin surface during pulse delivery,
- a pulse duration, and/or
- a location of the focus, preferably with respect to the main ultrasound propagation axis)

15. The HITU device (200) according to any one of the preceding claims, further adapted to automatically select, based on a detected occurrence of a hyperechoic mark, and preferably its geometrical parameters (location, area...), and preferably taking into consideration at least one other parameter, at least one parameter selected from the following group:

- a movement speed of the focus,
- a duty cycle of the high-power radiofrequency signal,
- a pulse repetition frequency of the high-power radiofrequency signal,
- a frequence band of the high-power radiofrequency signal,
- a output power of the RF generator, and/or
- a instantaneous intensity at the skin surface during pulse delivery,
- a location of the focus, preferably along the main ultrasound propagation axis.

16. The HITU device (200) according to any one of the preceding claims, wherein the controller (211) is adapted to compute a dragging of a treated tissue and further to model a real displacement of the therapy transducer (202) considering the dragging, and preferably to set pulse parameters abased on the real displacement.

17. A method of treating a patient with HITU, including a step of emitting a HITU pulse (P) from a therapy transducer (202) onto a target, preferably a vein (V), wherein the therapy transducer (202) is moved in a first direction (215), while a HITU pulse (P) is being emitted.

18. The method according to claim 17, wherein the first direction (215) is contained in a plane containing a central axis of the vein (V).

19. The method according to any one of claims 17 to 18, wherein the first direction (215) is oriented substantially orthogonally with respect to a main ultrasound propagation axis and/or parallel to the skin of the patient.

20. The method according to any one of the claims 17 to 18, wherein the first direction (215) is oriented substantially parallel to the main ultrasound propagation axis and/or orthogonally to the skin of the patient.

21. The method according to any one of claims 17 to 20, wherein the motion of the focus (F) is a rotational motion around an acoustic focus.

22. The method according to any one of the claims 17 to 21 wherein the motion of the focus (F) follows a vein (V) border along a targeted segment.

23. The method according to any one of the claims 17 to 22, further comprising at least one, preferably all, of the following steps:

- placing the focus on a center of the vein, or placing the focus on a right or a left, with respect to an ultrasound image, extremity of the vein during a targeting phase when the motion of the focus is done along an axis which is substantially orthogonal with respect to the main ultrasound propagation axis,

- the focus is moved to a position outside a tissue area to be treated before emission of the pulse, preferably outside each of a sequence of tissue areas to be treated such as to account for an acceleration phase during which no pulse is emitted,

- at the end of the sequence of pulses and/or when the sequence is interrupted, moving the focus back to the center of the vein and/or to an initial position of the focus, and/or remain at a final position of a last motion.

24. The method according to any one of the claims 17 to 23, further comprising synchronizing the emission of the pulse and motion of the focus.

25. The method according to 24, wherein the synchronization is such that a smaller equivalent power is emitted when the motion is decelerated.

26. The method according to any one of claims 24 or 25, wherein the synchronizing is such that smaller equivalent power is emitted when the motion is accelerated.

**Fig. 1a**

**Fig. 1b**

Fig. 1c

**Fig. 2**

**Fig. 3**

42

43

41

Fig. 4

54

53

51

52

55

Fig. 5

**Fig. 6**

**Fig. 7**

**Fig. 8**

**Fig. 9**

102

101

104

105

103

**Fig. 10**

112

111

115

117

114

116

113

**Fig. 11**

a       b       c       d

121

122

123

124

125

**Fig. 12**

a        b        c        d

131

132

133

134

135

**Fig. 13**

143

142

141

**Fig. 14**

152

151

**Fig. 15**

**Fig. 16**

Europäisches
Patentamt
European
Patent Office
Office européen
des brevets

# EUROPEAN SEARCH REPORT

Application Number

EP 25 31 5018

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/273246 A1 (DARLINGTON GREGORY P [US] ET AL) 1 October 2015 (2015-10-01) | 1-5,16 | INV.<br>A61N7/02 |
| Y | * paragraphs [0001], [0068], [0069], | 6-15 | A61B34/00 |
| A | [0089], [0092] *<br>* figures 6C,7 * | 17-26 | A61B8/00 |
| | ----- | | ADD. |
| X | US 2022/305298 A1 (ANQUEZ JÉRÉMIE [FR] ET AL) 29 September 2022 (2022-09-29)<br>* paragraphs [0001], [0077] *<br>* figure 2 * | 1,2 | A61B90/00<br>A61N7/00<br>A61B34/10 |
| | ----- | | |
| Y | US 2011/208055 A1 (DALAL SANDEEP [US] ET AL) 25 August 2011 (2011-08-25) | 6-8,14, 15 | |
| A | * paragraphs [0039], [0040], [0044] *<br>* figures 3-5 * | 4,5 | |
| | ----- | | |
| Y | US 2006/264748 A1 (VAEZY SHAHRAM [US] ET AL) 23 November 2006 (2006-11-23)<br>* paragraphs [0062] - [0064] *<br>* figures 8-9B * | 9-11 | |
| | ----- | | TECHNICAL FIELDS SEARCHED (IPC) |
| Y | US 2012/238873 A1 (LACOSTE FRANCOIS [FR] ET AL) 20 September 2012 (2012-09-20)<br>* paragraph [0072] * | 12,13 | A61N<br>A61B |
| | ----- | | |

The present search report has been drawn up for all claims

| Place of search | Date of completion of the search | Examiner |
|---|---|---|
| The Hague | 27 June 2025 | Milles, Julien |

EPO FORM 1503 03.82 (P04C01)

## ANNEX TO THE EUROPEAN SEARCH REPORT
## ON EUROPEAN PATENT APPLICATION NO.

EP 25 31 5018

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2025

| Patent document cited in search report | Publication date | Patent family member(s) | Publication date |
|---|---|---|---|
| US 2015273246 A1 | 01-10-2015 | CA 2795166 A1 | 06-10-2011 |
| | | CN 103025381 A | 03-04-2013 |
| | | CN 107050674 A | 18-08-2017 |
| | | EP 2552546 A2 | 06-02-2013 |
| | | JP 5805176 B2 | 04-11-2015 |
| | | JP 2013523287 A | 17-06-2013 |
| | | KR 20130094695 A | 26-08-2013 |
| | | US 2010241005 A1 | 23-09-2010 |
| | | US 2015273246 A1 | 01-10-2015 |
| | | US 2018015309 A1 | 18-01-2018 |
| | | WO 2011123862 A2 | 06-10-2011 |
| US 2022305298 A1 | 29-09-2022 | US 2022305298 A1 | 29-09-2022 |
| | | WO 2020254419 A1 | 24-12-2020 |
| US 2011208055 A1 | 25-08-2011 | BR PI0914520 A2 | 15-12-2015 |
| | | CN 102202735 A | 28-09-2011 |
| | | EP 2352558 A1 | 10-08-2011 |
| | | JP 5948057 B2 | 06-07-2016 |
| | | JP 2012507324 A | 29-03-2012 |
| | | RU 2011122671 A | 20-12-2012 |
| | | US 2011208055 A1 | 25-08-2011 |
| | | WO 2010052596 A1 | 14-05-2010 |
| US 2006264748 A1 | 23-11-2006 | AT E493103 T1 | 15-01-2011 |
| | | AU 2005284746 A1 | 23-03-2006 |
| | | CA 2576068 A1 | 23-03-2006 |
| | | EP 1789136 A2 | 30-05-2007 |
| | | JP 4755186 B2 | 24-08-2011 |
| | | JP 2008513148 A | 01-05-2008 |
| | | US 2006264748 A1 | 23-11-2006 |
| | | WO 2006032058 A2 | 23-03-2006 |
| US 2012238873 A1 | 20-09-2012 | CN 102781516 A | 14-11-2012 |
| | | EP 2332614 A1 | 15-06-2011 |
| | | EP 2512599 A1 | 24-10-2012 |
| | | KR 20120123034 A | 07-11-2012 |
| | | KR 20170120215 A | 30-10-2017 |
| | | US 2012238873 A1 | 20-09-2012 |
| | | WO 2011069985 A1 | 16-06-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020254419 A1
- US 20170151448 A1
- US 9770605 B2
- EP 3164192 A
- US 9028471 B2

**Non-patent literature cited in the description**

- **N BARNAT et al.** Noninvasive vascular occlusion with HIFU for venous insufficiency treatment: preclinical feasibility experience in rabbits. *Physics in Medicine and Biology*, 2019, vol. 64 (2), 025003